# EUROPEAN PATENT APPLICATION

(11) **EP 4 755 871 A1**
(43) Date of publication of application: **10.06.2026**
(21) Application number: 24849216.7
(22) Date of filing: 31.07.2024
(51) Int. Cl.: C07C 67/055, C07B 61/00, C07C 69/15

(54) **METHOD FOR PRODUCING VINYL ACETATE**

(30) Priority: 01.08.2023 JP 2023125311
(71) Applicant: Kuraray Co., Ltd., Kurashiki-shi, Okayama 710-0801 (JP)
(72) Inventor: KAWAHIRA, Yuta, La Porte, Texas 77571 (US); KANNO, Takehiro, Okayama-shi, Okayama 702-8601 (JP); JIKIHARA, Atsushi, Kurashiki-shi, Okayama 710-0801 (JP)
(74) Representative: Müller-Boré & Partner Patentanwälte PartG mbB
(86) International application number: PCT/JP2024/027371
(87) International publication number: WO 2025/028571

(57) **Abstract**

A method for producing vinyl acetate, the method including at least: a step (1) of reacting ethylene, acetic acid and oxygen to obtain a crude product containing vinyl acetate; a step (2) of separating a gaseous composition containing ethylene and vinyl acetate from the crude product; and a step (3) of introducing at least a part of the gaseous composition into a gas washing column, and removing vinyl acetate from the gaseous composition, wherein the gas washing column has at least an outlet (A) and an outlet (B); a total content of acetic acid and water in a liquid composition (α) to be taken out from the outlet (A) is 70 mass% or more; a total content of vinyl acetate and acetic acid in a liquid composition (β) to be taken out from the outlet (B) is 90 mass% or more and a content of water in the liquid composition (β) is 10 mass% or less. Accordingly, in a method for producing vinyl acetate, energy required for separating vinyl acetate, water, and acetic acid by distillation is reduced.

## Description

### Technical Field

The present invention relates to a method for producing vinyl acetate. Particularly, the present invention relates to a method for producing vinyl acetate, the method including at least: a step (1) of reacting ethylene, acetic acid and oxygen to obtain a crude product containing vinyl acetate; a step (2) of separating the crude product into a liquid composition containing vinyl acetate, acetic acid and water, and a gaseous composition containing ethylene and vinyl acetate; and a step (3) of introducing at least a part of the gaseous composition into a gas washing column, and washing the gaseous composition with acetic acid and water to remove vinyl acetate from the gaseous composition.

### Background Art

Vinyl acetate is a useful compound used for such as a raw material of polyvinyl acetate. As an industrial method for producing the same, the Wacker process is widely adopted, and vinyl acetate is produced using ethylene, acetic acid and oxygen as raw materials.

Patent Literature 1 discloses an example of a specific production method by the Wacker process, and describes a method for producing vinyl acetate in a continuous gas phase process using a heterogeneous catalyst by reacting ethylene with acetic acid and oxygen in a reactor, and
a) separating the product gas stream comprising essentially ethylene, vinyl acetate, acetic acid, water, carbon dioxide and further inert gases, and
b) recycling an ethylene- and CO₂-containing cycle gas stream into the reactor, by
c) compressing the cycle gas stream in a cycle gas compressor before recycling into the reactor, and
d) branching off a substream of the cycle gas on the suction side or the pressure side of the cycle gas compressor and feeding it to a CO₂ scrubbing, and
e) prior to the CO₂ scrubbing, washing it in a water scrubber, wherein
f) the substream, after the CO₂ scrubbing, is supplied to the cycle gas via a jet compressor, together with ethylene as a jet gas, on the pressure side of the cycle gas compressor and downstream of the withdrawal of the substream to the CO₂ scrubbing, and/or
g) a bottom product from the water scrubber is fed directly to the preliminary dewatering column.

By subjecting the substream of the cycle gas to CO₂ scrubbing, CO₂ by-produced in the vinyl acetate synthesis reaction is prevented from accumulating in the cycle gas. Then, the gas before being subjected to the CO₂ scrubbing is previously passed through the water scrubber and washed with water and acetic acid to remove vinyl acetate in the gas. When the gas is washed in this way, a bottom product containing water, acetic acid and vinyl acetate is generated at the bottom of the water scrubber. The bottom product is distilled together with the reaction crude product containing vinyl acetate to obtain vinyl acetate from which water and acetic acid have been separated.

### Citation List

### Patent Literature

Patent Literature 1: JP 2012-524757 A

### Summary of Invention

### Technical Problem

When vinyl acetate is produced by the method described in Patent Literature 1, thermal energy such as steam is required to separate acetic acid, water and vinyl acetate by distillation. In particular, since energy required to evaporate a large amount of water is large, measures for reducing the energy have been demanded.

The present invention has been devised to solve this problem, and an object of the present invention is to reduce energy required for separating vinyl acetate, water and acetic acid by distillation in a method for producing vinyl acetate.

### Solution to Problem

The above problem is solved by the following inventions.
[1] A method for producing vinyl acetate, the method comprising at least:
   a step (1) of reacting ethylene, acetic acid and oxygen to obtain a crude product containing vinyl acetate;
   a step (2) of separating the crude product into a liquid composition containing vinyl acetate, acetic acid and water, and a gaseous composition containing ethylene and vinyl acetate; and
   a step (3) of introducing at least a part of the gaseous composition into a gas washing column, and washing the gaseous composition with acetic acid and water to remove vinyl acetate from the gaseous composition, wherein
   the gas washing column has at least an outlet (A) and an outlet (B),
   a liquid composition (α) to be taken out from the outlet (A) contains acetic acid and water, and a total content of acetic acid and water in the liquid composition (α) is 70 mass% or more, and
   a liquid composition (β) to be taken out from the outlet (B) contains vinyl acetate and acetic acid, a total content of vinyl acetate and acetic acid in the liquid composition (β) is 90 mass% or more and a content of water in the liquid composition (β) is 10 mass% or less.
[2] The production method according to [1], wherein the outlet (A) is positioned above the outlet (B).
[3] The production method according to [1] or [2], wherein the gas washing column has an acetic acid inlet and a water inlet, and the outlet (A) is positioned above the acetic acid inlet and below the water inlet.
[4] The production method according to any one of [1] to [3], further comprising a step (4) of introducing the liquid composition (α) into an extraction column, extracting acetic acid with vinyl acetate, and separating a liquid composition containing vinyl acetate and acetic acid and a liquid composition containing water.
[5] The production method according to [4], wherein in the extraction column, the liquid composition (α) and vinyl acetate are brought into countercurrent contact, and the liquid composition containing vinyl acetate and acetic acid and the liquid composition containing water are separated.
[6] The production method according to [5], wherein the liquid composition (α) is introduced from an upper part of the extraction column, vinyl acetate is introduced from a lower part of the extraction column, the liquid composition (α) and the vinyl acetate are brought into countercurrent contact, the liquid composition containing vinyl acetate and acetic acid is taken out from an upper part of the extraction column, and the liquid composition containing water is taken out from a lower part of the extraction column.
[7] The production method according to any one of [1] to [6], further comprising a step (5) of introducing the liquid composition separated in the step (2) into a distillation column and distilling the liquid composition.
[8] The production method according to [7], further comprising a step of introducing the liquid composition (β) into the distillation column.
[9] The production method according to [7] or [8], further comprising a step of introducing the liquid composition containing vinyl acetate and acetic acid separated in the step (4) into the distillation column.
[10] The production method according to any one of [7] to [9], wherein vinyl acetate, acetic acid, and water are separated in the distillation column.
[11] The production method according to [10], wherein vinyl acetate obtained in the step (5) is used in the step (4) after being optionally purified.
[12] The production method according to any one of [1] to [11], wherein
   in the step (3), the gaseous composition is washed with acetic acid and water to separate the gaseous composition into a gaseous composition containing ethylene and carbon dioxide and a liquid composition containing vinyl acetate,
   the production method further comprises a step (6) of introducing the gaseous composition containing ethylene and carbon dioxide into a carbon dioxide removal system to separate the gaseous composition into ethylene and carbon dioxide, and
   the ethylene separated in the step (6) is used in the step (1).

### Advantageous Effects of Invention

According to the production method of the present invention, it is possible to reduce energy required for separating vinyl acetate, water and acetic acid by distillation, and it is possible to provide an industrial method for producing vinyl acetate with a small amount of energy used.

### Brief Description of Drawings

FIG. 1 is a schematic view of a vinyl acetate production apparatus used in Example 1.
FIG. 2 is a schematic view of a vinyl acetate production apparatus used in Comparative Example 1.

### Description of Embodiments

The present invention relates to a method for producing vinyl acetate. The present invention is a method for producing vinyl acetate, the method comprising at least:
a step (1) of reacting ethylene, acetic acid and oxygen to obtain a crude product containing vinyl acetate;
a step (2) of separating the crude product into a liquid composition containing vinyl acetate, acetic acid and water, and a gaseous composition containing ethylene and vinyl acetate; and
a step (3) of introducing at least a part of the gaseous composition into a gas washing column, and washing the gaseous composition with acetic acid and water to remove vinyl acetate from the gaseous composition, wherein
the gas washing column has at least an outlet (A) and an outlet (B),
a liquid composition (α) to be taken out from the outlet (A) contains acetic acid and water, and a total content of acetic acid and water in the liquid composition (α) is 70% or more, and
a liquid composition (β) to be taken out from the outlet (B) contains vinyl acetate and acetic acid and a total content of vinyl acetate and acetic acid in the liquid composition (β) is 90 mass% or more.

In the production method of the present invention, vinyl acetate is produced by the Wacker process. Thereby, vinyl acetate is synthesized in accordance with the following formula (I) using ethylene, acetic acid and oxygen as raw materials.

C₂H₄ + CH₃COOH + 1/2O₂ → CH₂CHOCOCH₃ + H₂O (I)

The step (1) is a step of reacting ethylene, acetic acid and oxygen to obtain a crude product containing vinyl acetate. In a reactor, a vinyl acetate synthesis reaction represented by the above formula (I) proceeds by a gas phase process using a heterogeneous catalyst. While ethylene, acetic acid and oxygen are brought under a pressure of 0.1 to 3 MPa at a temperature of 130 to 200°C into contact with a fixed bed catalyst containing palladium or a palladium compound and an alkali metal salt on a carrier material, vinyl acetate and water are produced. The reaction is an exothermic reaction, and vinyl acetate is continuously synthesized. At this time, a side reaction represented by the following formula (II) also proceeds, and a part of ethylene is oxidized by oxygen to generate carbon dioxide. As a result, the crude product taken out of the reactor contains vinyl acetate and water, which are reaction products, ethylene, acetic acid and oxygen, which are substances unreacted, and carbon dioxide, which is a by-product.

C₂H₄ + 3O₂ → 2CO₂ + 2H₂O (II)

The step (2) is a step of separating the crude product obtained in the step (1) into a liquid composition containing vinyl acetate, acetic acid and water and a gaseous composition containing ethylene and vinyl acetate. The liquid composition may contain a small amount of other components. In addition to ethylene and vinyl acetate, carbon dioxide and oxygen are usually also contained in the gaseous composition, and water, acetic acid, or the like may further be contained in a gaseous state. The separation device for separating the liquid composition and the gaseous composition is not particularly limited, but it is preferable to continuously separate the liquid composition and the gaseous composition using a vertical gas separation column. In this case, it is preferable to feed acetic acid to the separation device, thereby dissolving vinyl acetate and water in acetic acid, and take a liquid composition containing vinyl acetate, acetic acid and water out of the separation device. Specifically, it is preferable to feed acetic acid from an upper part of the vertical separation column, and take the liquid composition out of a bottom part. It is also preferable that the method further includes a step of introducing the liquid composition thus separated in the step (2) into a distillation column.

The step (3) is a step of introducing at least a part of the gaseous composition obtained in the step (2) into a gas washing column, and washing the gaseous composition with acetic acid and water to remove vinyl acetate from the gaseous composition. By removing the vinyl acetate, it is possible to prevent a decrease in the carbon dioxide removal ability in the carbon dioxide removal system of the step (6) described later. The whole amount of the gaseous composition obtained in the step (2) may be subjected to the step (3), but it is sufficient that the concentration of the carbon dioxide by-produced in the step (1) is controlled not to excessively increase, and thus only a part of the gaseous composition may be introduced into a gas washing column and feed it to the carbon dioxide removal system. Usually, it is sufficient to introduce 1 to 20 mass% of the gaseous composition obtained in the step (2) into the gas washing column.

The gas washing column has two outlets, namely, an outlet (A) and an outlet (B), and may further have another outlet. Liquid compositions after being used for gas washing are taken out from both of the outlet (A) and the outlet (B). The liquid composition (α) is taken out from the outlet (A). The liquid composition (α) contains acetic acid and water, and the total content of acetic acid and water in the liquid composition (α) is 70 mass% or more. The total content of acetic acid and water is preferably 80 mass% or more, and more preferably 90 mass% or more. The liquid composition (α) mainly contains vinyl acetate in addition to acetic acid and water. On the other hand, the liquid composition (β) is taken out from the outlet (B). The liquid composition (β) contains vinyl acetate and acetic acid, and the total content of vinyl acetate and acetic acid in the liquid composition (β) is 90 mass% or more, and at this time, the content of water is 10 mass% or less. When the content of water is 10 mass% or less, the energy required when the liquid composition (β) is subjected to distillation can be reduced. The content of water is preferably 5 mass%, and more preferably 2 mass% or less. On the other hand, the total content of vinyl acetate and acetic acid is preferably 95 mass% or more, and more preferably 98 mass% or more.

In the gas washing column to be used in the step (3), the outlet (A) is preferably positioned above the outlet (B). This makes it possible to reduce the water content of the liquid composition (β) taken out from the outlet (B). Preferably, the gas washing column has an acetic acid inlet and a water inlet, and the outlet (A) is positioned above the acetic acid inlet and below the water inlet. This makes it possible to remove vinyl acetate in a gas by dissolving it in acetic acid, and also possible to remove acetic acid contained in the gas after the removal of the vinyl acetate by dissolving it in water. As a result, the liquid composition (α) containing acetic acid and water as main components is taken out from the outlet (A) positioned above the acetic acid inlet and below the water inlet. In addition, the liquid composition (β) containing vinyl acetate and acetic acid as main components is taken out from the outlet (B) placed below the outlet (A). The place where the gaseous composition obtained in the step (2) is introduced into the gas washing column is preferably below the acetic acid inlet in consideration of the efficiency of vinyl acetate dissolution. In addition, the outlet (B) is preferably provided below the place where the gaseous composition obtained in the step (2) is introduced into the gas washing column, and particularly preferably provided at the bottom of the column. On the other hand, the position where the gas washed in the gas washing column is taken out is preferably above the water inlet, and particularly preferably at the top of the column. That is, in the gas washing column, a post-washing gas outlet, the water inlet, the outlet (A), the acetic acid inlet, a pre-washing gas inlet, and the outlet (B) are preferably arranged in this order from the top toward the bottom. It is preferable that the method further includes a step of introducing the liquid composition (β) thus taken out from the outlet (B) in the step (3) into the distillation column.

It is preferable that the method further includes a step (4) of introducing the liquid composition (α) taken out from the outlet (A) of the gas washing column in the step (3) into an extraction column, extracting acetic acid with vinyl acetate, and separating a liquid composition containing vinyl acetate and acetic acid and a liquid composition containing water. The extraction column to be used in the step (4) is not particularly limited as long as it is an extraction column in which liquid-liquid extraction can be performed. The liquid composition (α) and liquid vinyl acetate are introduced into the extraction column, and both come into contact with each other in a liquid phase. As a result, acetic acid is extracted with vinyl acetate from the liquid composition (α) mainly composed of water and acetic acid, and the liquid composition containing vinyl acetate and acetic acid and the liquid composition containing water are separated. It is preferable that in the extraction column, the liquid composition (α) and vinyl acetate are brought into countercurrent contact, and the liquid composition containing vinyl acetate and acetic acid and the liquid composition containing water are separated. Further, it is more preferable that the liquid composition (α) is introduced from an upper part of the extraction column, vinyl acetate is introduced from a lower part of the extraction column, the liquid composition (α) and the vinyl acetate are brought into countercurrent contact, the liquid composition containing vinyl acetate and acetic acid is taken out from an upper part of the extraction column, and the liquid composition containing water is taken out from a lower part of the extraction column. Since the specific gravity of an aqueous phase is higher than the specific gravity of an oil phase, this configuration is suitably employed. It is preferable that the method further includes a step of introducing the liquid composition containing vinyl acetate and acetic acid thus separated in the step (4) into a distillation column. The total content of vinyl acetate and acetic acid in the liquid composition containing vinyl acetate and acetic acid is preferably 80 mass% or more, more preferably 90 mass% or more, and still more preferably 95 mass% or more. The content of water in the liquid composition containing water is preferably 80 mass% or more, more preferably 90 mass% or more, and still more preferably 95 mass% or more.

It is preferable that the method further includes a step (5) of introducing the liquid composition separated in the step (2) into the distillation column and distilling the liquid composition. In this case, it is preferable to introduce the liquid composition (β) taken out from the outlet (B) in the step (3) into the distillation column, and it is also preferable to introduce the liquid composition containing vinyl acetate and acetic acid separated in the step (4) into the distillation column. Further, it is more preferable to subject the liquid composition separated in the step (2), the liquid composition (β), and the liquid composition separated in the step (4) to the step (5) of distilling them together. From a mixed liquid containing vinyl acetate, acetic acid and water, a vinyl acetate fraction, a water fraction and an acetic acid fraction are obtained by distillation. Each fraction may contain a small amount of other components. According to the preferred production method of the present invention, most of the water used for gas washing in the step (3) can be removed by the liquid-liquid extraction operation in the step (4). Therefore, it is possible to separate with little energy by phase separation between the aqueous phase and the oil phase, so that the amount of water evaporated by the distillation in the step (5) is small. As a result, the energy consumption can be reduced. When steam is used as the heat source, the amount of the steam can be reduced. It is also preferable that the vinyl acetate obtained in the step (5) is optionally purified and then used for extracting acetic acid in the step (4).

It is preferable that in the step (3), the gaseous composition is washed with acetic acid and water to separate the gaseous composition into a gaseous composition containing ethylene and carbon dioxide and a liquid composition containing vinyl acetate, and the method further includes a step (6) of introducing the gaseous composition containing ethylene and carbon dioxide into a carbon dioxide removal system to separate the gaseous composition into ethylene and carbon dioxide. It is also preferable that the ethylene thus separated in the step (6) is introduced into the reactor of the step (1) and used in a reaction of vinyl acetate synthesis. When a part of the gaseous composition let out of the separation device of step (2) is subjected to step (3) and subsequent step (6), it is preferable that the ethylene separated in the step (6) is merged with the rest of the gaseous composition let out of the separation device of the step (2), further ethylene, acetic acid and oxygen are added, and then the resultant mixture is introduced into the reactor of the step (1) and used in the reaction of vinyl acetate synthesis. By recycling a gaseous composition containing unreacted raw materials as described above, vinyl acetate can be efficiently produced without waste.

### Examples

### [Example 1]

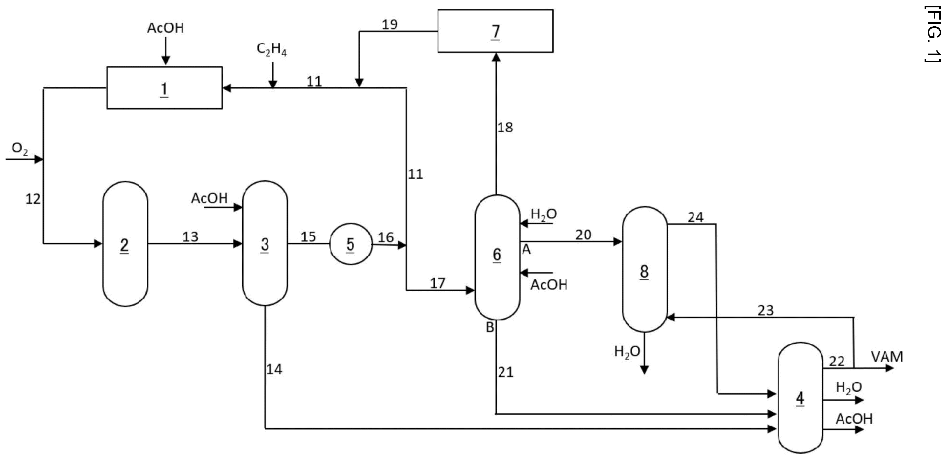

Vinyl acetate was produced with an apparatus shown in FIG. 1. A circulated gas containing ethylene was introduced into an acetic acid evaporator 1 from a conduit 11, and acetic acid vaporized in the acetic acid evaporator 1 was mixed with the circulated gas and let out of a conduit 12. Oxygen was fed to the circulated gas in the conduit 12 and then introduced into a reactor 2. In the reactor 2, a vinyl acetate synthesis reaction was proceeded, and then a crude product was taken out into a conduit 13. The crude product contained vinyl acetate and water generated through the reaction in the reactor 2, ethylene, acetic acid and oxygen, which were substances unreacted, and carbon dioxide, which was a by-product.

The crude product was introduced into a gas separation column 3 from the conduit 13, and liquid acetic acid was introduced from the upper part of the column and brought into contact with gas. Thus, a liquid composition containing vinyl acetate, acetic acid and water and a gaseous composition containing ethylene, oxygen, carbon dioxide, vinyl acetate, acetic acid and water were separated. The resulting liquid composition was introduced into a distillation column 4 via a conduit 14. In addition, the gaseous composition was introduced into a compressor 5 via a conduit 15, and the gaseous composition compressed by the compressor 5 was let out into a conduit 16. About 95 mass% and the remaining about 5 mass% of the gaseous composition were fed to the conduit 11 and a conduit 17, respectively, via the conduit 16.

About 5 mass% of the gaseous composition was introduced into a lower part of a gas washing column 6 from the conduit 17. In the gas washing column 6, water was fed through an inlet, and acetic acid was fed through an inlet provided below that inlet. A liquid composition (α) containing acetic acid and water was taken out into a conduit 20 through an outlet (A) positioned between the water inlet and the acetic acid inlet. In addition, a liquid composition (β) containing vinyl acetate and acetic acid was taken out into a conduit 21 through an outlet (B) positioned in a lower part of the gas washing column 6. Further, a gaseous composition containing ethylene, carbon dioxide and oxygen was taken out into a conduit 18 from an upper part of the gas washing column 6. The total content of acetic acid and water in the liquid composition (α) was about 100 mass%, and the total content of vinyl acetate and acetic acid in the liquid composition (β) was about 99 mass% and the content of water was about 1 mass%. The gas washing column 6 had an outlet for the gaseous composition, the water inlet, the outlet (A), the acetic acid inlet, an inlet for the gaseous composition, and the outlet (B) in this order from the top.

The gaseous composition taken out of the gas washing column 6 was introduced into a CO₂ removal system 7 via the conduit 18, the carbon dioxide was removed and then introduced into the conduit 11, and further ethylene was introduced into the conduit 11 and then returned to the acetic acid evaporator 1. That is, most of the gaseous composition is circulated from the acetic acid evaporator 1 through the conduits 12, 13, 15, 16, 11 to the acetic acid evaporator 1. The remainder of the gaseous composition is circulated through the conduits 17, 18, 19, 11 and is returned to the acetic acid evaporator 1 with carbon dioxide removed. As a result, it is possible to prevent carbon dioxide as a by-product from being accumulated in the circulated gas and allowing an increase of the concentration thereof.

The liquid composition (β) taken out from the gas washing column 6 was introduced into the distillation column 4 via the conduit 21. In addition, the liquid composition (α) taken out from the gas washing column 6 was introduced into an upper part of an acetic acid extraction column 8 via the conduit 20. The acetic acid extraction column 8 was a column-type liquid-liquid extraction apparatus, and had an outlet for a liquid composition containing vinyl acetate and acetic acid, an inlet for the liquid composition (α), an inlet for vinyl acetate, and an outlet for a liquid composition containing water in this order from the top.

The liquid composition (α) containing acetic acid and water was fed through the conduit 20 to an upper part of the acetic acid extraction column 8, and vinyl acetate (liquid) was fed through a conduit 23 to a lower part of the acetic acid extraction column 8. The vinyl acetate is one obtained from the distillation column 4. The density of the liquid composition (α) was higher than the density of vinyl acetate, so that the liquid composition (α) descended, the vinyl acetate ascended, and the liquid composition (α) and the vinyl acetate came into countercurrent contact with each other in the acetic acid extraction column 8. Since acetic acid was more soluble in vinyl acetate than in water, acetic acid was dissolved in the ascending vinyl acetate. As a result, a liquid composition containing vinyl acetate and acetic acid was taken out from the upper part of the acetic acid extraction column 8 and fed to the distillation column 4 via a conduit 24. In addition, a liquid composition containing water was taken out from the lower part of the acetic acid extraction column 8. The total content of vinyl acetate and acetic acid in the liquid composition containing vinyl acetate and acetic acid was about 99 mass%, and the content of water in the liquid composition containing water was about 97 mass%.

A liquid composition containing vinyl acetate, acetic acid and water was introduced into the distillation column 4 via the conduits 14, 21, and 24, and the vinyl acetate, the acetic acid and the water were separated from each other by distillation. The vinyl acetate obtained by the distillation was taken out through the conduit 22, and a part thereof was fed to the acetic acid extraction column 8 via the conduit 23. During continuous operation, the amount of steam required in the distillation column 4 was 16.2 ton/h.

### [Comparative Example 1]

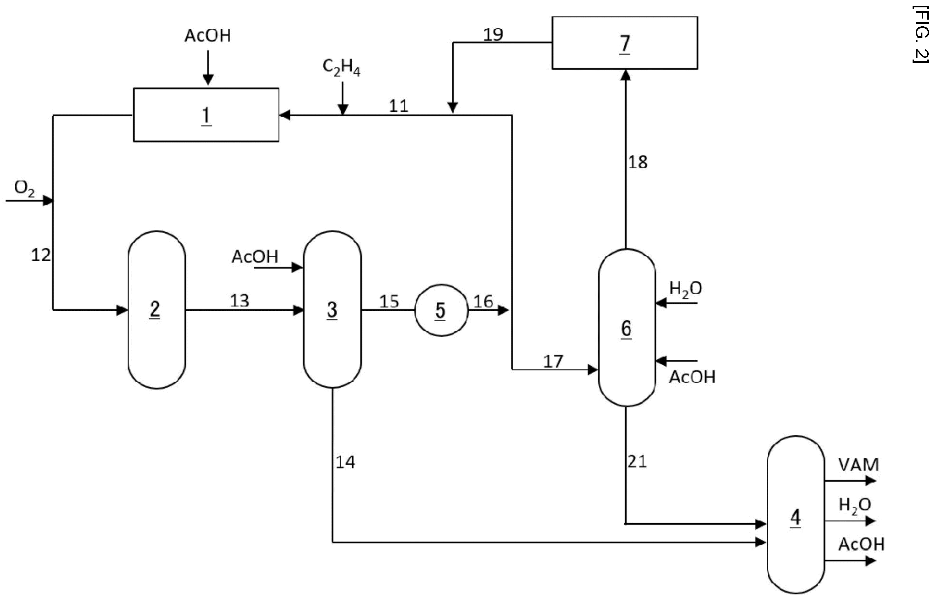

Vinyl acetate was produced with an apparatus shown in FIG. 2. Vinyl acetate was continuously produced using an apparatus having the same design as in Example 1 except that the acetic acid extraction column 8 was not provided. Since the acetic acid extraction column 8 was not provided, the conduit 20 connecting the gas washing column 6 and the acetic acid extraction column 8, the conduit 24 connecting the acetic acid extraction column 8 and the distillation column 4, and the conduit 23 connecting the conduit 22 and the acetic acid extraction column 8 were not provided.

In the gas washing column 6, as in Example 1, water and acetic acid were fed through the respective inlets, and a gaseous composition was introduced into the CO₂ removal system 7 via the conduit 18. On the other hand, the whole amount of a liquid composition obtained in the gas washing column 6 was taken out into the conduit 21 through an outlet positioned in the lower part of the gas washing column 6. The liquid composition contained vinyl acetate, acetic acid and water, the total content of vinyl acetate and acetic acid was about 85 mass%, and the content of water was about 15 mass%. The gas washing column 6 had an outlet for the gaseous composition, a water inlet, an acetic acid inlet, an inlet for the gaseous composition, and an outlet for the liquid composition in this order from the top. The feed amounts of water and acetic acid were the same as those in Example 1.

The liquid composition was introduced into the distillation column 4 through the outlet of gas washing column 6 via the conduit 21. In addition, a liquid composition was introduced into the distillation column 4 from the gas separation column 3 via the conduit 14. Distillation was performed in the distillation column 4 to separate vinyl acetate, acetic acid and water. During continuous operation, the amount of steam required in the distillation column 4 was 18.0 ton/h. Since the amount of water to be separated in the distillation column 4 was large, more steam was needed than in Example 1.

### Reference Signs List

- 1: acetic acid evaporator
- 2: reactor
- 3: gas separation column
- 4: distillation column
- 5: compressor
- 6: gas washing column
- 7: CO₂ removal system
- 8: acetic acid extraction column
- 11 to 24: conduit

## Claims

1. A method for producing vinyl acetate, the method comprising at least:
a step (1) of reacting ethylene, acetic acid and oxygen to obtain a crude product containing vinyl acetate;
a step (2) of separating the crude product into a liquid composition containing vinyl acetate, acetic acid and water, and a gaseous composition containing ethylene and vinyl acetate; and
a step (3) of introducing at least a part of the gaseous composition into a gas washing column, and washing the gaseous composition with acetic acid and water to remove vinyl acetate from the gaseous composition, wherein
the gas washing column has at least an outlet (A) and an outlet (B),
a liquid composition (α) to be taken out from the outlet (A) contains acetic acid and water, and a total content of acetic acid and water in the liquid composition (α) is 70 mass% or more, and
a liquid composition (β) to be taken out from the outlet (B) contains vinyl acetate and acetic acid, a total content of vinyl acetate and acetic acid in the liquid composition (β) is 90 mass% or more and a content of water in the liquid composition (β) is 10 mass% or less.

2. The production method according to claim 1, wherein the outlet (A) is positioned above the outlet (B).

3. The production method according to claim 1 or 2, wherein the gas washing column has an acetic acid inlet and a water inlet, and the outlet (A) is positioned above the acetic acid inlet and below the water inlet.

4. The production method according to claim 1 or 2, further comprising a step (4) of introducing the liquid composition (α) into an extraction column, extracting acetic acid with vinyl acetate, and separating a liquid composition containing vinyl acetate and acetic acid and a liquid composition containing water.

5. The production method according to claim 4, wherein in the extraction column, the liquid composition (α) and vinyl acetate are brought into countercurrent contact, and the liquid composition containing vinyl acetate and acetic acid and the liquid composition containing water are separated.

6. The production method according to claim 5, wherein the liquid composition (α) is introduced from an upper part of the extraction column, vinyl acetate is introduced from a lower part of the extraction column, the liquid composition (α) and the vinyl acetate are brought into countercurrent contact, the liquid composition containing vinyl acetate and acetic acid is taken out from an upper part of the extraction column, and the liquid composition containing water is taken out from a lower part of the extraction column.

7. The production method according to claim 1 or 2, further comprising a step (5) of introducing the liquid composition separated in the step (2) into a distillation column and distilling the liquid composition.

8. The production method according to claim 7, further comprising a step of introducing the liquid composition (β) into the distillation column.

9. The production method according to claim 7, further comprising a step of introducing the liquid composition containing vinyl acetate and acetic acid separated in the step (4) into the distillation column.

10. The production method according to claim 7, wherein vinyl acetate, acetic acid, and water are separated in the distillation column.

11. The production method according to claim 10, wherein vinyl acetate obtained in the step (5) is used in the step (4) after being optionally purified.

12. The production method according to claim 1 or 2, wherein
in the step (3), the gaseous composition is washed with acetic acid and water to separate the gaseous composition into a gaseous composition containing ethylene and carbon dioxide and a liquid composition containing vinyl acetate,
the production method further comprises a step (6) of introducing the gaseous composition containing ethylene and carbon dioxide into a carbon dioxide removal system to separate the gaseous composition into ethylene and carbon dioxide, and
the ethylene separated in the step (6) is used in the step (1).
